(19) Europäisches Patentamt
European Patent Office
Office européen des brevets

(11) **EP 2 960 705 B1**

(12) **EUROPÄISCHE PATENTSCHRIFT**

(45) Veröffentlichungstag und Bekanntmachung des Hinweises auf die Patenterteilung:
**06.03.2024   Patentblatt 2024/10**

(21) Anmeldenummer: **15172224.6**

(22) Anmeldetag: **16.06.2015**

(51) Internationale Patentklassifikation (IPC):
**A61B 3/13** (2006.01)     **G02B 21/12** (2006.01)
**G02B 21/08** (2006.01)

(52) Gemeinsame Patentklassifikation (CPC):
**A61B 3/13; G02B 21/082; G02B 21/125**

(54) **BELEUCHTUNGSVORRICHTUNG FÜR EIN OPTISCHES BEOBACHTUNGSGERÄT**

ILLUMINATING DEVICE FOR AN OPTICAL OBSERVATION UNIT

DISPOSITIF D'ÉCLAIRAGE POUR UN APPAREIL D'OBSERVATION OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.06.2014   DE 102014212371**

(43) Veröffentlichungstag der Anmeldung:
**30.12.2015   Patentblatt 2015/53**

(73) Patentinhaber: **Carl Zeiss Meditec AG**
**07745 Jena (DE)**

(72) Erfinder:
• **Reimer, Peter**
**73479 Ellwangen (DE)**
• **Kolster, Daniel**
**73447 Oberkochen (DE)**
• **Merz, Franz**
**73433 Aalen (DE)**
• **Meinkuß, Stefan**
**89564 Nattheim (DE)**

(74) Vertreter: **Carl Zeiss AG - Patentabteilung**
**Carl-Zeiss-Straße 22**
**73447 Oberkochen (DE)**

(56) Entgegenhaltungen:
DE-A1- 10 304 267      DE-A1- 19 856 847
DE-A1-102012 217 967   JP-A- H0 868 942
US-A1- 2010 302 630    US-A1- 2012 057 013

**Beschreibung**

Die Erfindung betrifft ein Operationsmikroskop mit einer Beleuchtungsvorrichtung, die eine Beleuchtungslichtquelle mit unabhängig voneinander steuerbaren Einzellichtquellen, die in einer ersten Ebene in einem zweidimensionalen Array angeordnet sind, und einen Beleuchtungsstrahlengang mit einer Beleuchtungsoptik und einer optischen Achse umfasst. Durch die Beleuchtungsoptik ist eine zu der ersten Ebene konjugierten Ebene gebildet.

Die JP H08 68942 A offenbart eine Beleuchtungsvorrichtung für ein Mikroskop, die mehrere Lichtquellen und eine Schalteinrichtung für die Lichtquellen aufweisen kann.

Die US 2012/057013 A1 offenbart eine Visualisierungsvorrichtung, die mehrere Lichtquellen aufweisen kann.

Aus der US 2010/302630 A1 ist eine Beleuchtungsvorrichtung für ein Mikroskop bekannt, die eine Lichtquelle mit wenigstens zwei zweidimensionalen lichtemittierenden Segmenten aufweist.

Aus der DE 103 04 267 A1 ist ein Augenchirurgie-Mikroskopsystem bekannt, das eine Hintergrund-Beleuchtungseinrichtung zum Erzeugen einer Rotreflex-HintergrundBeleuchtung aufweist.

Aus der DE 198 56 847 A1 ist ein Operationsmikroskop bekannt, das sowohl die Betrachtung der roten Reflexion als auch des Schattenkontrastes ermöglicht.

Die DE 10 2012 217 967 A1 offenbart ein Mikroskop zur rasterfreien, konfokalen Abbildung einer in einem Probenraum angeordneten Probe.

Aus der DE 10 2006 013 761 A1 ist eine Beleuchtungseinrichtung mit einer Lichtquelle, die aus einer Anordnung von mehreren schaltbaren Kleinstlichtquellen gebildet ist, bekannt.

Nachteilig an dieser Beleuchtungseinrichtung ist, dass für unterschiedliche Beleuchtungsvarianten nicht in jedem Fall eine optimale Beleuchtungsqualität erreichbar ist. Beim Einbringen einer starren Blende in den Beleuchtungsstrahlengang ist üblicherweise die Blendenöffnung an die maximale Größe der Lichtquelle angepasst, um eine scharfe Abbildung der Lichtquelle in einem zu beleuchtenden Objektbereich zu erreichen. Bei einer Verkleinerung der Lichtquellengröße ist dann eine Abbildung der Lichtquelle, insbesondere der Ränder der Lichtquelle, in dem zu beleuchtenden Objektbereich unschärfer. Mögliche Bildfehler einer Beleuchtungsoptik können sich stärker auswirken. Streulicht oder Reflexlicht, das durch die Beleuchtungsoptik oder durch Fassungsränder optischer Elemente entsteht, wird in den Objektbereich abgebildet. Die Beleuchtungsqualität wird insgesamt schlechter.

Beim Einsatz einer variablen Blende kann die Größe einer Blendenöffnung an die Größe der Beleuchtungslichtquelle angepasst werden. Der Aufwand für eine variable Blendenöffnung und deren Steuerung ist jedoch hoch. Die Verwendung von transmissiven Blenden, beispielsweise Flüssigkristalldisplays, ist technisch aufwendig und führt zu Lichtverlusten. Diese ist nachteilig bei kleinen Lichtquellen mit begrenzter Lichtleistung.

Es ist daher eine Aufgabe der Erfindung, eine verbesserte Beleuchtungsvorrichtung für ein optisches Beobachtungsgerät sowie ein Verfahren zu deren Betrieb bereitzustellen, mit denen bei unterschiedlichen Beleuchtungsvarianten eine hohe Beleuchtungsqualität erreichbar ist.

Die Aufgabe wird erfindungsgemäß durch ein Operationsmikroskop mit den Merkmalen des unabhängigen Anspruchs 1 und ein Verfahren mit den Merkmalen des unabhängigen Patentanspruchs 13 gelöst. Vorteilhafte Weiterbildungen der Erfindung sind in den Unteransprüchen beschrieben.

Erfindungsgemäß umfasst das Operationsmikroskop eine Blendeneinrichtung, die in der zu der ersten Ebene konjugierten Ebene angeordnet ist. Die Blendeneinrichtung weist eine Anzahl von Blendenöffnungen auf, wobei jede Blendenöffnung einer Einzellichtquelle zugeordnet ist.

Die Beleuchtungslichtquelle umfasst unabhängig voneinander steuerbare Einzellichtquellen, die in einem Array in einer ersten Ebene angeordnet sind. Die Anordnung der Einzellichtlichtquellen in einem Array ermöglicht einen kompakten Aufbau der Beleuchtungslichtquelle. Durch eine voneinander unabhängige Steuerung der Einzellichtquellen lassen sich sehr schnell unterschiedliche Beleuchtungsvarianten einstellen. Das Array der unabhängig steuerbaren Einzellichtquellen wird durch eine Beleuchtungsoptik in eine zu der ersten Ebene konjugierten Ebene abgebildet, in der eine Blendeneinrichtung angeordnet ist. Die Blendeneinrichtung weist eine Anzahl von Blendenöffnungen auf, wobei jeder Einzellichtquelle eine einzelne Blendenöffnung zugeordnet ist. Somit kann für jede Einzellichtquelle eine hohe Abbildungsqualität in einer Objektebene erreicht werden. Jede steuerbare Einzellichtquelle kann mit einem scharf begrenzten Rand in eine Objektebene abgebildet werden. Mögliche Bildfehler der Beleuchtungsoptik wirken sich nur gering aus, so dass vorteilhaft eine kostengünstige Beleuchtungsoptik verwendet werden kann. Streulicht oder Reflexlicht, das durch die Beleuchtungsoptik oder durch Fassungsränder optischer Elemente entsteht, wird vorteilhaft durch die Blendeneinrichtung ausgeblendet oder deutlich verringert. Die Beleuchtungsqualität ist bei jeder Beleuchtungsvariante sehr hoch.

Durch die einzelne Ansteuerung der Einzellichtquellen lassen sich nacheinander verschiedene Beleuchtungsvarianten sehr schnell bereitstellen, in dem die Einzellichtquellen einzeln oder kombiniert ein-/ausgeschaltet oder in der Helligkeit individuell angesteuert werden. Die Beleuchtungsvarianten sind somit in Form, Größe und Helligkeit unterschiedlich einstellbar. Vorteilhaft kann eine Anpassung des Beleuchtungslichtes durch die einzeln steuerbaren Einzellichtquellen an wechselnde Anwendungssituationen erfolgen.

Die Größe und Form der Blendenöffnungen sind an Bilder der Einzellichtquellen in der konjugierten Ebene angepasst. Eine erste Hauptebene (H) und eine zweite Hauptebene (H') ist durch die Beleuchtungsoptik definiert. Ein erster Abstand s ist als Abstand zwischen der ersten Hauptebene (H) und der ersten Ebene und ein zweiter Abstand s' als Abstand zwischen der zweiten Hauptebene (H') und der zu der ersten Ebene konjugierten Ebene gebildet. Für einen Vergrößerungsmaßstab ist folgende Beziehung erfüllt: $1 \leq |\frac{s'}{s}| \leq 4$, bevorzugt $1.3 \leq |\frac{s'}{s}| \leq 3$, besonders bevorzugt $1.6 \leq |\frac{s'}{s}| \leq 2.3$

Die Einzellichtquellen können sehr klein sein. Durch die Anpassung der Blendenöffnungen in Größe und Form an Bilder der Einzellichtquellen in der konjugierten Ebene in einem bestimmten Vergrößerungsmaßstab kann die Blendeneinrichtung einfacher und kostengünstiger produziert werden. Fertigungstoleranzen der Blendenöffnungen wirken sich geringer aus. Bei dem genannten Vergrößerungsmaßstab ist bei sehr guter Beleuchtungsstärke eine sehr gute Abbildungsleistung erreichbar. Dies resultiert in einer sehr hohen Beleuchtungsqualität bei sehr guter Ergonomie.

In einer Ausgestaltung der Erfindung weist die Beleuchtungslichtquelle mindestens vier Einzellichtquellen auf.

Durch mindestens vier Einzellichtquellen sind mindestens drei verschiedene Beleuchtungsvarianten einstellbar.

In einer Ausgestaltung der Erfindung sind in dem zweidimensionalen Array nicht alle Positionen durch Einzellichtquellen besetzt.

Jeder Blendenöffnung ist eine Einzellichtquelle zugeordnet. Sind in dem zweidimensionalen Array nicht alle Positionen durch Einzellichtquellen besetzt, kann vorteilhaft auf die jeweils zugeordnete Blendenöffnung verzichtet werden. Die Blende kann günstiger produziert werden. Durch die nicht besetzte Position wird kein Streulicht verursacht. Eine mögliche Wärmeentwicklung der Beleuchtungseinheit ist geringer.

In einer Ausgestaltung der Erfindung sind die Einzellichtquellen quadratisch, rechteckig oder rund ausgeführt.

Die Ausführung der Einzellichtquellen in quadratischen, rechteckig oder runden Form ermöglicht eine einfache Herstellung der Blendenöffnungen, die in Größe und Form an die Einzellichtquellen angepasst sind. Eine Fertigung eines Arrays mit Einzellichtquellen der oben genannten Form ist kostengünstig.

In einer Ausgestaltung der Erfindung sind die in dem zweidimensionalen Array angeordneten Einzellichtquellen kreuzförmig angeordnet.

Damit ist eine Beleuchtung in zwei Achsrichtungen möglich. Bei beispielsweise vier Einzellichtquellen kann eine Einzellichtquelle auf jedem Schenkel der kreuzförmigen Anordnung positioniert sein.

In einer Ausgestaltung der Erfindung sind die in dem zweidimensionalen Array angeordneten Einzellichtquellen in mindestens zwei Zeilen und mindestens zwei Spalten angeordnet.

Bei dieser Anordnung sind vielfältige Beleuchtungsvarianten einstellbar. Durch eine unabhängige Steuerung von Einzellichtquellen einzeln oder in Kombination, in Spalten oder in Zeilen sind Beleuchtungswinkel und Helligkeiten variierbar.

In einer Ausgestaltung der Erfindung ist eine Steuerungsvorrichtung vorhanden, durch die die Helligkeit jeder Einzellichtquelle durch Spannungs- und/oder Stromsteuerung oder durch eine Pulsweitenmodulation mit frei einstellbaren Pulsverhältnissen steuerbar ist.

Die Beleuchtungsvarianten sind durch das spezifische Steuern oder Regeln einzelner Einzellichtquellen an bestimmten Positionen des zweidimensionalen Arrays einstellbar. Durch das Zuschalten einer oder mehrerer Einzellichtquellen kann die Helligkeit des Beleuchtungslichtes erhöht werden. Vorteilhaft kann die Helligkeit der Einzellichtquellen zusätzlich durch eine Steuerung der Spannung und/oder des Stroms definiert werden. Um die Einzellichtquellen in einem breiten Helligkeitsbereich in sehr feinen Abstufungen einstellen zu können, kann vorteilhafte eine Steuerung durch Pulsweitenmodulation mit frei einstellbaren Pulsverhältnissen erfolgen.

In einer Ausgestaltung der Erfindung ist die Steuerungsvorrichtung derart ausgebildet, dass eine oder mehrere Einzellichtquellen mit geringerer oder höherer Intensität oder mit einem anderen Frequenzmuster als die übrigen Einzellichtquellen ansteuerbar sind, so dass diese eine oder mehrere Einzellichtquellen ein Fixierlicht bilden.

Ein Fixierlicht ist ein Lichtpunkt, den ein Patient während einer Operation mit seinem Auge fixiert. Damit befindet sich die Lage des Patientenauges in einer definierten und stabilen Position. Das Fixierlicht kann durch eine einzeln ansteuerbare Einzellichtquelle oder mehrerer Einzellichtquellen dem Patientenauge angeboten werden. Es ist keine zusätzliche Optik notwendig. Das Fixierlicht wird durch die Beleuchtungsoptik auf die Netzhaut eines rechtsichtigen Patientenauges scharf abgebildet. Vorteilhaft können bereits vorhandene Komponenten einer Steuerungsvorrichtung zur Steuerung der Einzellichtquellen ebenfalls für die Steuerung des Fixierlichtes eingesetzt werden. Ein Fixierlicht ist damit platzsparend und kostengünstig realisierbar.

In einer Ausgestaltung der Erfindung ist eine oder mehrere Einzellichtquellen zur Abstrahlung von Licht einer anderen Wellenlänge als die übrigen Einzellichtquellen ausgebildet.

Unterscheidet sich eine Einzellichtquelle in der abgestrahlten Wellenlänge, bzw. in der Farbe von den als Beleuchtungslicht eingesetzten Einzellichtquellen, ist es für ein zu untersuchende Auge sehr schnell und leicht erkennbar und kann vorteilhaft ein Fixierlicht bilden.

Bei der Ausgestaltung des Operationsmikroskops können vorteilhaft verschieden Beleuchtungsvarianten realisiert werden. Typischerweise wird das Beleuchtungslicht in einen ersten monoskopischen oder stereoskopischen Beobachtungsstrahlengang eingekoppelt. Auch die Einkopplung in einen zweiten monoskopischen oder stereoskopischen Beobachtungsstrahlengang ist vorstellbar. Durch die Anordnung der Einzellichtquellen in einem zweidimensionalen Array können durch eine selektive Steuerung der Einzellichtquellen unterschiedliche Beleuchtungswinkel erreicht werden. Durch eine koaxiale Einkopplung eines Beleuchtungsstrahlenganges in einen Beobachtungsstrahlengang kann vorteilhaft eine Rotreflex-Beleuchtung gebildet werden.

In einer Ausgestaltung der Erfindung ist in einem Beobachtungsstrahlengang eine Kamera angeordnet ist, die mit einer Steuerungsvorrichtung verbunden ist. Die Steuerungsvorrichtung ist zur Detektion eines Rotreflexes eines Auges und zur Ansteuerung der in dem zweidimensionalen Array angeordneten Einzellichtquellen in Abhängigkeit von einer Maßzahl für den Rotreflex ausgebildet.

Damit kann eine automatische Steuerung der Einzellichtquellen abhängig von einer Beleuchtungssituation eines zu beobachtenden Auges erfolgen. Die Bilder einer Kamera sind durch die Steuerungsvorrichtung in Bezug auf einen Rotreflex des Auges auswertbar. Abhängig von der Ausbildung des Rotreflexes ermittelt die Steuerung eine Maßzahl. Eine Beleuchtungsvariante kann abhängig von der berechneten Maßzahl automatisch eingestellt werden.

Gemäß einem weiteren Aspekt der Erfindung wird ein Verfahren zur automatischen Umschaltung einer Beleuchtungsvariante eines Operationsmikroskops bereitgestellt, gekennzeichnet durch die Schritte:

- Aufnahme eines Bildes eines Auges durch die in dem Beobachtungsstrahlengang angeordnete Kamera;
- Auswerten des Bildes der Kamera durch die Steuerungsvorrichtung;
- Detektieren eines Rotreflexes des Auges durch die Steuerungsvorrichtung;
- Bestimmen einer Maßzahl für den Rotreflex;
- Ansteuern der in einem zweidimensionalen Array angeordneten Einzellichtquellen in Abhängig der Maßzahl für den Rotreflex.

Ein Rotreflex in einem Auge kann durch Auswertung der Bilder einer im Beobachtungsstrahlengang angeordneten Kamera detektiert werden. Abhängig von der Ausbildung des Rotreflexes ermittelt die Steuerung eine Maßzahl. Abhängig von der Maßzahl erfolgt eine automatische Ansteuerung einer einzelnen oder mehrere Einzellichtquellen. Somit kann eine automatisierte Einstellung oder Umschaltung einer Beleuchtungsvariante für das Operationsmikroskop bereitgestellt werden.

Weitere Vorteile und Merkmale der Erfindung werden in Bezug auf die nachfolgenden Zeichnungen erklärt, in welchen zeigen:

Figur 1     ein erstes Ausführungsbeispiel eines erfindungsgemäßen Operationsmikroskops mit einer Beleuchtungsvorrichtung;

Figur 2     ein Ausführungsbeispiel für eine Lichtquelleneinheit mit einem Einzellichtquellen-Array;

Figur 3     das Einzellichtquellen-Array gemäß Figur 2 in einer schematischen Darstellung;

Figur 4     acht verschiedene Beleuchtungsvarianten des Einzellichtquellen-Arrays gemäß Figur 3;

Figur 5     ein Ausführungsbeispiel einer Blendeneinrichtung;

Figur 6     eine schematische Darstellung eines Strahlengangs zwischen der Lichtquelleneinheit und der Blendeneinrichtung gemäß Figur 1;

Figur 7     eine Abbildung der Beleuchtungsspots auf einem Fundus eines Auges für einen stereoskopischen Beobachter;

Figur 8     eine Abbildung der Beleuchtungsspots auf einem Fundus für zwei stereoskopische Beobachter;

Figur 9     ein Auge, das auf ein Fixierlicht ausgerichtet ist.

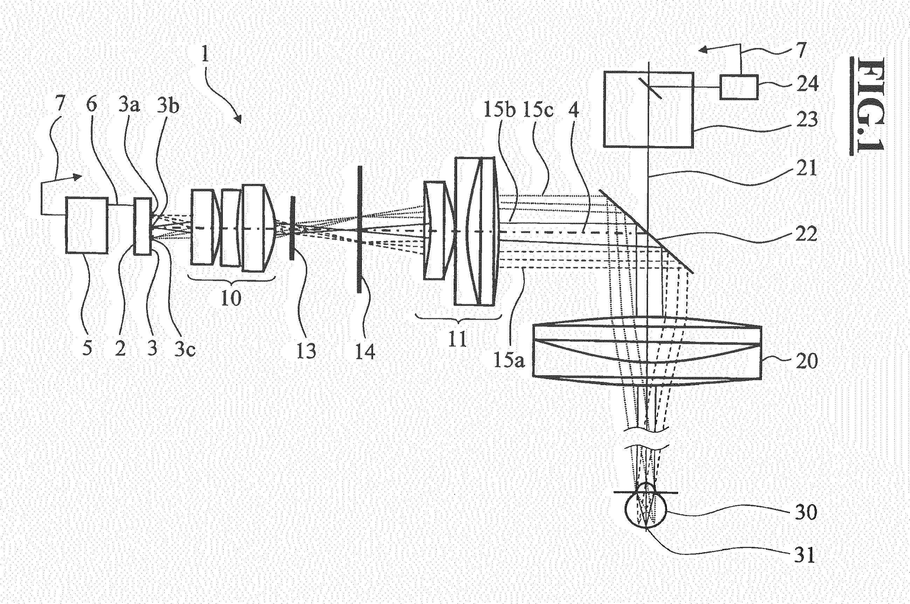

Figur 1 zeigt ein erstes Ausführungsbeispiel eines erfindungsgemäßen Operationsmikroskops mit einer Beleuchtungsvorrichtung 1. Das Operationsmikroskop ist als Stereomikroskop ausgebildet.

Die Beleuchtungsvorrichtung 1 weist eine Lichtquelleneinheit 2, eine erste Linsengruppe 10, eine Leuchtfeldblende 13, eine Blendeneinrichtung 14 und eine zweite Linsengruppe 11 auf, die entlang einer optischen Achse 4 angeordnet sind. Die Lichtquelleneinheit 2 umfasst ein zweidimensionales Einzellichtquellen-Array 3, welches eine erste Einzellichtquelle 3a, eine zweite Einzellichtquelle 3b und eine dritte Einzellichtquelle 3c aufweist. Das Einzellichtquellen-Array 3 umfasst weitere, nicht dargestellte Einzellichtquellen. Das Einzellichtquellen-Array 3 ist in einer ersten Ebene senkrecht zur optischen Achse 4 angeordnet. Die zweite Einzellichtquelle 3b ist auf der optischen Achse 4 oder angeordnet. Die Lichtquelleneinheit 2 ist über eine Leitung 6 mit einer Steuerungsvorrichtung 5 verbunden.

Die von dem Einzellichtquellen-Array 3 erzeugten Beleuchtungslichtstrahlenbündel werden durch die erste Linsengruppe 10, die Leuchtfeldblende 13, die Blendeneinrichtung 14 und die zweite Linsengruppe 11 geführt und über ein Umlenkelement 22, beispielsweise einen Strahlteiler, in einen Beobachtungsstrahlengang des Operationsmikroskops eingekoppelt. Das Operationsmikroskop weist ein Hauptobjektiv 20 und eine nur schematisch angedeutete Beobachtungsoptik 23 auf. Ein stereoskopische Beobachtungsstrahlengang für einen ersten Beobachter ist parallel zu einer optischen Achse 21 ausgebildet. In diesem Ausführungsbeispiel ist der stereoskopische Beobachtungsstrahlengang mit einem linken und einen rechten Beobachtungsstrahlengang senkrecht zu der dargestellten Bildebene angeordnet, so dass die beiden optischen Achsen des linken und rechten Beobachtungsstrahlengangs in dieser Ansicht deckungsgleich sind.

An einen Beobachtungsstrahlengang ist über einen Strahlteiler eine Kamera 24 angekoppelt, die über eine Leitung 7 mit der Steuerungsvorrichtung 5 verbunden ist. An die Steuerungsvorrichtung kann eine nicht dargestellte Ein-Ausgabe-Einheit angeschlossen sein.

Die erste Linsengruppe 10 kann eine Einzellinse oder mehrere Linsenelemente aufweisen. Die erste Linsengruppe 10 bildet eine Kollektoroptik. Die erste Linsengruppe 10 bildet das Einzellichtquellen-Array 3 in eine zu der ersten Ebene konjugierten Ebene ab, in der die Blendeneinrichtung 14 angeordnet ist. Die Blendeneinrichtung 14 weist eine Anzahl von Blendenöffnungen auf, wobei jede Blendenöffnung einer Einzellichtquelle zugeordnet ist. Die Blendenöffnungen der Blendeneinrichtung 14 bilden dabei ein vergrößertes Abbild des Einzellichtquellen-Arrays 3. Die Blendeneinrichtung 14 ist in Bezug auf das Einzellichtquellen-Array 3 um 180° gedreht bezüglich der optischen Achse 4 angeordnet.

Die zweite Linsengruppe 11 bildet zusammen mit dem Hauptobjektiv 20 eine Kondensor-Linsengruppe. Die zweite Linsengruppe 11 kann eine Einzellinse oder eine Anzahl von Linsenelementen umfassen. Das Beleuchtungslicht ist unterhalb des Hauptobjektives 20 in einem parallelen Strahlenbündel geführt und wird nach Unendlich abgebildet. Das Beleuchtungslicht trifft auf ein zu beobachtendes Objekt, ein Auge 30. In dem Auge 30 wird das Lichtquellenbild der Einzellichtquellen auf einem Fundus 31 abgebildet.

Von der zweiten Einzellichtquelle 3b verläuft ein zweites Beleuchtungsstrahlenbündel 15b parallel zu der optischen Achse 4 und wird koaxial in den linken Beobachtungsstrahlengang eingekoppelt. Auf dem Fundus 31 des Auges 30 erzeugt das Licht der zweiten Einzellichtquelle 3b einen Beleuchtungsspot. Das vom Fundus 31 zurückgestreute Licht kann ein Beobachter beim Blick durch das Operationsmikroskop als Rotreflex wahrnehmen.

Wird der linke Beobachtungsstrahlengang auf den Fundus projiziert, bildet dieser einen in etwa runden Querschnitt auf dem Fundus. Der von der zweiten Einzellichtquelle 3b erzeugte Beleuchtungsspot liegt innerhalb dieses Querschnitts.

Ein von der ersten Einzellichtquelle 3a verlaufendes erstes Beleuchtungsstrahlenbündel 15a wird unter einem kleinen Winkel von ca. -2° bezüglich der optischen Achse eines Beobachtungsstrahlengangs zu dem Auge 30 geführt und bildet eine -2°-Beleuchtung.

Ein von der dritten Einzellichtquelle 3c verlaufendes drittes Beleuchtungsstrahlenbündel 15c wird dagegen unter einem kleinen Winkel von ca. 2° bezüglich der optischen Achse eines Beobachtungsstrahlengangs zu dem Auge 30 geführt und bildet eine 2°-Beleuchtung bezüglich der gegenüberliegenden Seite der optischen Achse.

Die oben beschriebenen Ausführungen gelten sowohl für einen linken als auch einen rechten Beobachtungsstrahlengang. Das Einzellichtquellen-Array 3 weist für den rechten Beobachtungsstrahlengang drei weitere, in der Bildebene hinter den Einzellichtquellen 3a, 3b, 3c liegende Einzellichtquellen auf.

Die Lichtquelleneinheit 2 umfasst das Einzellichtquellen-Array 3, wobei jede Einzellichtquelle aus einer LED oder einer OLED gebildet ist. Es ist auch vorstellbar, dass die Einzellichtquellen Lichtleitfasern umfassen und die Einzellichtquellen aus den Faserenden der Lichtleitfasern gebildet sind. Damit ist eine sehr kompakte Bauform der Einzellichtquellen erreichbar. Bei der Ausführung der Einzellichtquellen als LED's oder OLED's können die Einzellichtquellen sehr schnell und einfach elektrisch gesteuert werden. Bei der Ausgestaltung als Austrittsende von Lichtleitfasern wird in der Ebene des zweidimensionalen Arrays keine Wärme erzeugt und es kann an dieser Stelle auf wärmeableitende Maßnahmen verzichtet werden. Das Einzellichtquellen-Array 3 kann auch eine Kombination aus LED's, OLED's und Glasfasern umfassen. Es ist auch vorstellbar, dass die Einzellichtquellen durch Laser oder angeregte Konverter gebildet sind. Die Einzellichtquellen können eine quadratische, rechteckige oder runde Abstrahlfläche haben.

Die Steuerungsvorrichtung 5 ist dazu ausgebildet, die erste Einzellichtquellen 3a, die zweite Einzellichtquelle 3b, die dritte Einzellichtquelle 3c und alle weitere, nicht dargestellte Einzellichtquellen des Einzellichtquellen-Arrays unabhängig voneinander ein- und auszuschalten. Des Weiteren kann die Helligkeit aller Einzellichtquellen durch eine Steuerung der Spannung oder des Stroms der Einzellichtquellen eingestellt werden. Alternativ ist die Helligkeit durch eine Pulsweitenmodulation mit frei einstellbaren Pulsverhältnissen steuerbar. Eine elektronische Helligkeitseinstellung der Einzellichtquellen hat den Vorteil, dass keine beweglichen Teile, wie beispielsweise Filter in den Beleuchtungsstrahlengang eingebracht werden müssen.

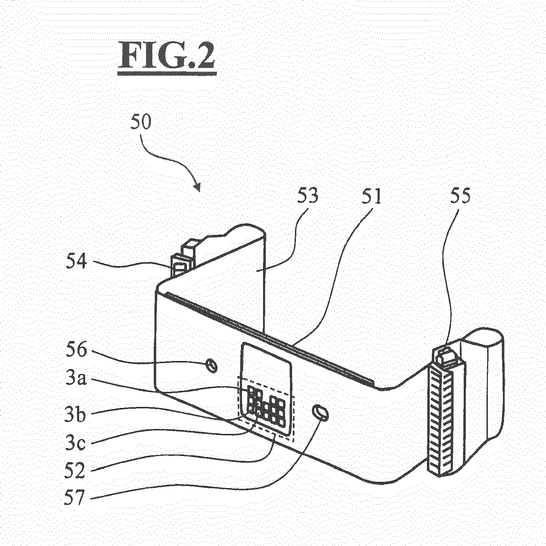

Figur 2 zeigt ein Ausführungsbeispiel einer Lichtquelleneinheit 50 mit einem Einzellichtquellen-Array. Das Einzellichtquellen-Array ist als LED-Matrix 52 mit vierzehn LED-Einzellichtquellen ausgebildet. Die LED-Einzellichtquellen sind als SMD-LED's auf einer Platine 51 angeordnet und können jeweils einzeln angesteuert werden. Die Anoden aller Einzellichtquellen können verbunden sein, während die Emitter jeweils über einzelne Leitung zu der Steuerungsvorrichtung 5 geführt sind. Es ist aber auch vorstellbar, dass die LED-Einzellichtquellen elektrisch in einer Gitteranordnung angeschlossen sind und über eine Multiplex-Elektronik angesteuert werden. Die Multiplex-Elektronik kann auf der Platine 51 angeordnet sein oder in der Steuerungseinheit 5 integriert sein. Die Platine 51 weist eine erste Aussparung 56 und eine zweite Aussparung 57 für jeweils ein mechanisches Befestigungselement auf. Die Platine ist mit einem Flachbandkabel 53 verbunden. Das Flachbandkabel weist einen ersten Steckverbinder 54 und einen zweiten Steckverbinder 55 auf, mit der die LED-Matrix 52 an die Steuerungsvorrichtung 5 angeschlossen werden kann.

Drei in einer Spalte der LED-Matrix 52 angeordneten LED-Einzellichtquellen können die in Figur 1 dargestellte erste Einzellichtquelle 3a, zweite Einzellichtquelle 3b und dritte Einzellichtquelle 3c bilden.

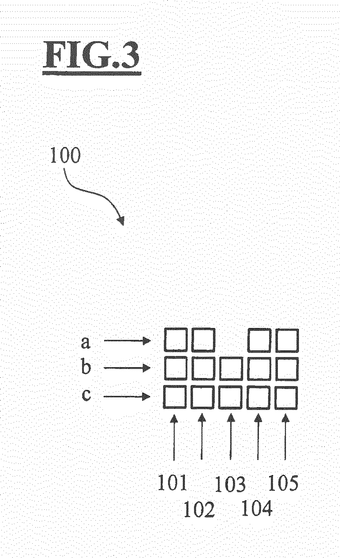

Figur 3 zeigt das Einzellichtquellen-Array gemäß Figur 2 in einer schematischen Darstellung. Die Anordnung der Einzellichtquellen entspricht der LED-Matrix 52. Die Einzellichtquellen eines Einzellichtquellen-Arrays 100 sind in drei Zeilen und fünf Spalten angeordnet. Die Zeilen sind mit Kleinbuchstaben und die Spalten mit Ziffern bezeichnet. Das Einzellichtquellen-Array weist insgesamt 14 Einzellichtquellen auf, wobei in einer ersten Zeile a vier Einzellichtquellen, in einer zweiten Zeile b und einer dritten Zeile c jeweils fünf Einzellichtquellen angeordnet sind. Eine erste Spalte 101, eine zweite Spalte 102, eine vierte Spalte 104 und eine fünfte Spalte 105 weisen jeweils drei Einzellichtquellen auf. In einer dritten Spalte 103 sind nur zwei Einzellichtquellen angeordnet.

Durch die Kombination der Ziffer der jeweiligen Spalte und des Buchstabens der jeweiligen Zeile kann die Position einer Einzellichtquelle in dem Einzellichtquellen-Array eindeutig definiert werden.

Wird eine Einzellichtquelle an einer bestimmten Position nicht eingeschaltet, so kann auf diese Einzellichtquelle im Array verzichtet werden. In diesem Ausführungsbeispiel wird auf die Einzellichtquelle an der Position 103a (Spalte 103, Zeile a) verzichtet, um mögliche Reflexe einer Einzellichtquelle an dieser Position vom Hauptobjektiv in einen Beobachtungsstrahlengang zu vermeiden. Somit entfällt eine ansonsten notwendige Reflexblende. Das Weglassen oder die Deaktivierung dieser Einzellichtquelle verringert Streulicht und eine mögliche Wärmeentwicklung der Beleuchtungseinheit.

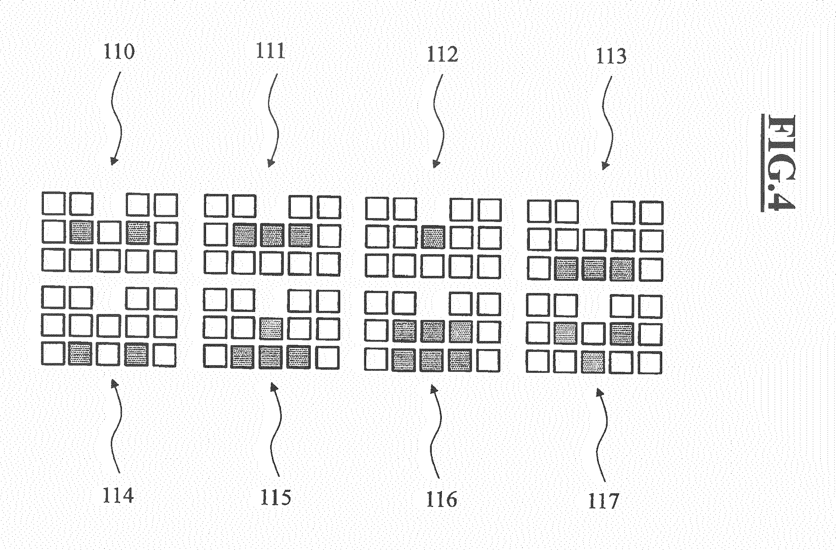

Figur 4 zeigt acht verschiedene Beleuchtungsvarianten des Einzellichtquellen-Arrays aus Figur 3. Durch die getrennte Ansteuerbarkeit jeder einzelnen Einzellichtquelle kann sowohl der Beleuchtungswinkel als auch der Durchmesser und die Helligkeit der Beleuchtung individuell an die jeweilige Situation bei der Beobachtung des Auges 30 oder den anatomischen Verhältnissen des Auges 30 angepasst werden.

In einer ersten Beleuchtungsvariante 110 sind zwei Einzellichtquellen an den Positionen 102b und 104b eingeschaltet. Das Licht dieser Einzellichtquellen ist jeweils koaxial in den linken und rechten Strahlengang der Beobachtungseinrichtung eingekoppelt. Die Beleuchtungsspots der Einzellichtquellen sind kleiner als die auf den Fundus projizierten Abbildungen des jeweils linken und rechten Beobachtungsstrahlenganges. Diese Beleuchtungsvariante 110 hat die Wirkung einer Stereo-Koaxialbeleuchtung. Der Beobachter kann durch die Beobachtungseinrichtung ein sehr kontrastreiches und homogenes Rotreflex-Bild wahrnehmen.

In einer zweiten Beleuchtungsvariante 111 ist zusätzlich zu den beiden Einzellichtquellen aus der Beleuchtungsvariante 110 noch eine dazwischenliegende Einzellichtquelle an der Position 103b eingeschaltet. Die zusätzliche Lichtquelle bewirkt eine Aufhellung des Rotreflexes. Der Kontrast des Rotreflexes ist gut, aber etwas geringer als in der ersten Beleuchtungsvariante 110.

In einer dritten Beleuchtungsvariante 112 ist nur die zentrale Einzellichtquelle an der Position 103b eingeschaltet. Ein Beobachter kann ein kontrastreiches Bild mit guter Tiefenwirkung jedoch bei geringer Helligkeit wahrnehmen. Es erfolgt nur ein geringer Lichteintrag in das Auge.

In einer vierten Beleuchtungsvariante 113 sind in der dritten Zeile c die drei mittleren Einzellichtquellen an den Positionen 102c, 103c, 104c aktiviert. Damit ist ein heller Rotreflex wie bei einer 2°-Beleuchtung zu erzielen. Die 2°-Beleuchtung bewirkt eine große Tiefenwirkung. Kontrast und Homogenität sind akzeptabel. Die zusätzlich eingeschaltete Einzellichtquelle an der Position 103c bewirkt eine Aufhellung des Bildes.

In einer fünften Beleuchtungsvariante 114 sind in der dritten Zeile c nur die zwei Einzellichtquellen an den Positionen 102c und 104c eingeschaltet. Damit ist ein guter Rotreflex wie bei einer 2°-Beleuchtung zu erzielen. Die 2°-Beleuchtung bewirkt eine große Tiefenwirkung bei akzeptabler Homogenität. Der Bildeindruck ist etwas dunkler als in der vierten Beleuchtungsvariante 113, dafür ist der Kontrast besser.

In einer sechsten Beleuchtungsvariante 115 leuchten insgesamt vier Einzellichtquellen, an den Positionen 103b, 102c, 103c, 104c. Diese Beleuchtungsvariante hat Wirkung einer 2°-Beleuchtung wie die vierte Beleuchtungsvariante 113. Die zusätzlich aktivierte Einzellichtquelle an den Positionen 103b führt zu einer Aufhellung des Bildes, jedoch bei etwas verringertem Kontrast.

In einer siebten Beleuchtungsvariante 116 sind insgesamt sechs Einzellichtquellen aktiviert, an den Positionen 102b, 103b, 104b, 102c, 103c, 104c. Diese Kombination stellte eine Kombination der zweiten Beleuchtungsvariante 111 und der vierten Beleuchtungsvariante 113 dar. Das Ergebnis ist ein sehr helles Bild. Der Kontrast ist jedoch gering.

In einer achten Beleuchtungsvariante 117 leuchten drei Einzellichtquellen. Zu den beiden Einzellichtquellen an den Positionen 102b und 104b aus der Beleuchtungsvariante 110 ist zusätzlich die Einzellichtquelle an der Position 103c eingeschaltet. Diese Beleuchtungsvariante hat die Wirkung einer Stereo-Koaxialbeleuchtung bei sehr gutem Kontrast und guter Homogenität und bei verbesserter Tiefenwirkung.

Eine Anwendung der beschriebenen Beleuchtungsvarianten sind beispielsweise in der Augenchirurgie, insbesondere der Kataraktchirurgie, von Bedeutung. In der ersten Phase einer Katarakt-Operation ist ein hoher Kontrast erwünscht, um den Verlauf einer Kapsulorhexis gut kontrollieren zu können. Vorteilhaft ist die ersten Beleuchtungsvariante 110 einer Stereo-Koaxialbeleuchtung aktiviert, die einen kleinen Fundusspot bewirkt. Der Beobachter kann durch die Beobachtungseinrichtung ein sehr kontrastreiches und homogenes Rotreflex-Bild wahrnehmen. Die Linsenkapsel des Auges ist durch den hohen Kontrast sehr gut erkennbar.

In einer zweiten Phase, der sogenannten Hydrodisektion und der Entfernung des Linsenkerns durch eine Phakoemulsifikation, wird die Struktur der Augenlinse stark verändert. Das Linsenmaterial bewirkt eine Streuung des Beleuchtungslichts, so dass nur noch wenig Beleuchtungslicht den Fundus erreicht. Der Rotreflex wird schwächer oder verschwindet ganz. In dieser zweiten Phase ist die Helligkeit der Beleuchtung zu erhöhen. Zudem ist ein größerer Fundusspot von Vorteil. Eine größere Tiefenschärfe kann durch eine 2°-Beleuchtung bewirkt werden. Eine 2°-Beleuchtung bildet die fünften Beleuchtungsvariante 114. Um die Helligkeit zu erhöhen, können weitere Einzellichtquellen eingeschaltet werden. Es bieten sich die vierte Beleuchtungsvariante 113 an, bei der eine weitere Einzellichtquelle eingeschaltet ist. Reicht die Helligkeit noch nicht aus, können zwei Einzellichtquellen in der sechsten Beleuchtungsvariante 115 oder vier Einzellichtquellen in der siebten Beleuchtungsvariante 116 zusätzlich aktiviert werden.

In einer dritten Phase, der Endphase der Phakoemulsifikation werden die letzten Reste der Augenlinse entfernt. Da die trüben und streuenden Linsenpartikel weitestgehend aus dem Auge entfernt sind, kann die Helligkeit der Beleuchtung verringert werden. Es ist ein hoher Kontrast gefordert, der bei einer koaxialen Beleuchtung mit kleinen Fundusspots erreicht wird. Es erfolgt die Aktivierung der erste Beleuchtungsvariante 110 oder der zweite Beleuchtungsvariante 111.

Auf diese Weise kann die für die jeweilige Einsatz-Phase optimierte Beleuchtungsvariante eingestellt werden. Der Beobachter kann zwischen einer Koaxialbeleuchtung oder einer Schrägbeleuchtung, bzw. 2° - Beleuchtung wechseln oder beide Beleuchtungsarten kombinieren. Die Größe der Fundusspots, der Beleuchtungswinkel und die Helligkeit kann dabei an die jeweilige Situation optimal angepasst werden.

In der Steuerungsvorrichtung 5 sind die verschiedene Beleuchtungsvarianten abgespeichert. Der Beobachter kann durch ein nicht dargestelltes Schaltelement die Beleuchtungsvarianten umschalten. Es ist vorstellbar, dass die Steuerungsvorrichtung ein Konfigurationsmenü aufweist, in der die verschiedenen Beleuchtungsvarianten voreinstellbar sind, die dann durch das Schaltelement im Betriebsmodus sequentiell aktiviert werden können. Das Schaltelement kann Teil eines Handgriffs, eines Fußschaltpultes, einer Ein-Ausgabe-Einheit oder eines anderen bekannten Eingabeelements sein.

Es ist auch vorstellbar, dass die Steuerungsvorrichtung 5 eine Bildverarbeitungseinheit umfasst, die geeignet ist, die Bilddaten einer im Beobachtungsstrahlengang angeordneten Kamera 24 auszuwerten. Die Bildverarbeitung kann derart ausgebildet sein, dass diese den Rotreflex automatisch detektiert und eine Maßzahl, beispielsweise einen Parameterwert des Rotreflexes bestimmt. Eine Maßzahl oder ein Parameterwert kann durch die Helligkeit, Größe, Form oder Kontrastwert des Rotreflexes ermittelbar sein.

Abhängig von der Maßzahl kann die Steuerungsvorrichtung 5 eine Beleuchtungsvariante automatisch einstellen. Es kann vorgesehen sein, dass bei einem plötzlichen Verschwinden des Rotreflexes durch Einschalten mindestens einer weiteren Einzellichtquelle sofort die Helligkeit erhöht wird. Es ist auch vorstellbar das in diesem Fall automatisch die siebte Beleuchtungsvariante 116 eingestellt wird.

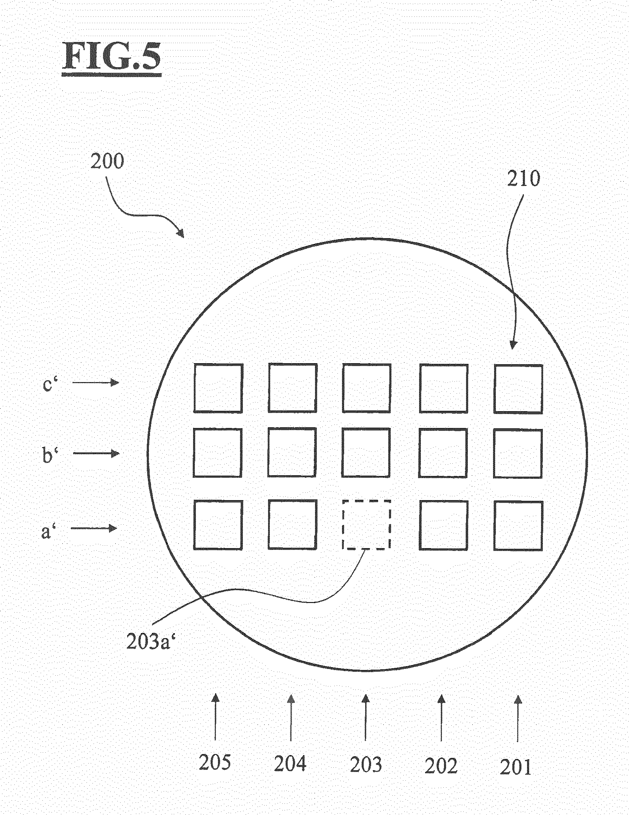

Figur 5 zeigt ein Ausführungsbeispiel einer Blendeneinrichtung 200. In diesem Ausführungsbeispiel ist die Blendeneinrichtung 200 kreisförmig ausgeführt. Die Blendeneinrichtung 200 weist Blendenöffnungen 210 auf, die einem vergrößerten Abbild des Einzellichtquellen-Arrays der Lichtquelleneinheit entsprechen. Figur 2 zeigt eine Ausführung der Lichtquelleneinheit als LED-Matrix 52 mit quadratisch ausgeführten Einzellichtquellen. Die Blendenöffnungen 201 sind deshalb ebenfalls quadratisch ausgeführt. Die Blendeneinrichtung 200 weist Blendenöffnungen 201 in drei Zeilen und fünf Spalten auf. Die Blendeöffnungen sind in einer ersten Zeile a', einer zweiten Zeile b' und einer dritten Zeile c', sowie einer ersten Spalte 201, einer zweiten Spalte 202, einer dritten Spalte 203, einer vierten Spalte 204 und einer fünften Spalte 205 angeordnet. Durch die Kombination der Ziffer der jeweiligen Spalte und des Buchstabens der jeweiligen Zeile kann die Position einer Blendenöffnung 210 eindeutig definiert werden.

Gemäß Figur 1 ist die Blendeneinrichtung 200 um 180° gedreht gegenüber des Einzellichtquellen-Arrays im Strahlengang entlang der optischen Achse angeordnet. Das Licht der Einzellichtquelle an der Position 102c gelangt somit durch die Blendenöffnung an der Position 202c'. Die Blendenöffnung an der Position 203a' ist optional. Bei dem Einzellichtquellen-Array gemäß der Figur 2 und Figur 3 ist aus Gründen der Reflexvermeidung auf die Einzellichtquelle an der Position 103a verzichtet worden. Deshalb ist in diesem Fall eine Blendenöffnung an der Position 203a' nicht notwendig. Beim Einsatz eines anderen Lichtquellen-Arrays, das eine Einzellichtquelle an der Position 103a aufweist, weist auch die Blendeneinrichtung 200 an der Position 203a' eine Blendenöffnung 210 auf.

Die Blendeneinrichtung 200 kann aus sehr dünnem Metallblech hergestellt sein. Die quadratischen Blendenöffnungen 210 können beispielsweise durch Stanzen, Laserschneiden, Wasserstrahlschneiden oder Ätzen hergestellt werden.

Die Blendenöffnungen 210 können auch rund ausgeführt sein. In diesem Fall korrespondieren die Mittelpunkte der runden Blendenöffnungen mit den Mittelpunkten der Einzellichtquellen.

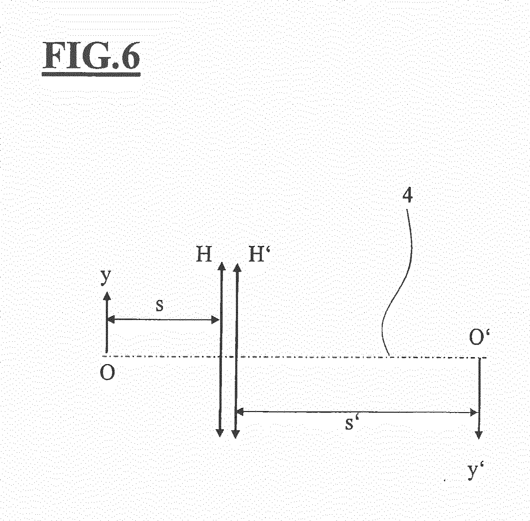

Figur 6 zeigt eine schematische Darstellung eines Strahlengangs zwischen der Lichtquelleneinheit 2 und der Blendeneinrichtung 14 gemäß Figur 1.

Die optische Achse 4 der Beleuchtungsoptik ist als Strichpunktlinie gezeichnet. Senkrecht zu der optischen Achse 4 befindet sich eine erste Hauptebene H als Bezugsebene für Brennweiten oder Abstandsangaben im Objektraum und eine zweite Hauptebene H' als Bezugsebene für den Bildraum. Die beiden Hauptebenen H, H' erlauben es, die Wirkung des erste Linsengruppe 10 mit der für eine dünne Linse gültigen Gleichung zu beschreiben. Die erste Hauptebene H und die zweite Hauptebene H' sind beide senkrecht zur optischen Achse des Linsensystems definiert und verlaufen damit parallel zueinander. Die beiden Hauptebenen H, H' ersetzen die erste Linsengruppe 10.

Eine erste Ebene ist durch das Einzellichtquellen-Array gebildet und ist senkrecht auf der optischen Achse 4 angeordnet. In dieser ersten Ebene liegt ein Objekt O. Der Objektpunkt O wird durch eine Einzellichtquelle des Einzellichtquellen-Arrays definiert und hat eine Objektgröße y. Der erste Abstand s ist der Abstand zwischen der ersten Hauptebene H und der ersten Ebene.

Der Objektpunkt O wird durch die erste Linsengruppe 10 zu einem Bildpunkt O' abgebildet. Der Bildpunkt O' liegt in einer zur ersten Ebene konjugierten Ebene. In dieser konjugierten Ebene ist die Blendeneinrichtung 14 angeordnet. Der zweite Abstand s' ist der Abstand zwischen der zweiten Hauptebene H' und der zu der ersten Ebene konjugierten Ebene mit dem Bildpunkt O'. Die Objektgröße y einer Einzellichtquelle wird in der konjugierten Ebene in vergrößertem Maßstab als Bildgröße y' dargestellt. Eine Blendenöffnung der Blendeneinrichtung 14, bzw. die gesamte Blendeneinrichtung 14, die einem Abbild des Einzellichtquellen-Array entspricht, ist deshalb in einem vergrößerten Maßstab ausgeführt. Für den Vergrößerungsmaßstab gilt folgende Beziehung: $1 \leq |\frac{s'}{s}| \leq 4$, bevorzugt $1.3 \leq |\frac{s'}{s}| \leq 3$, beson-

ders bevorzugt $1.6 \leq |\frac{s'}{s}| \leq 2.3$

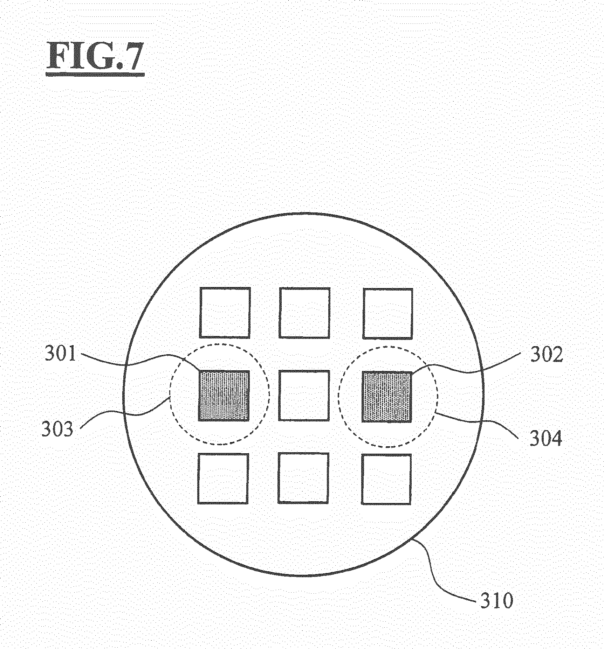

Figur 7 zeigt eine Abbildung der Beleuchtungsspots auf einem Fundus eines Auges für einen stereoskopischen Beobachter. In diesem Beispiel sind neun Einzellichtquellen des Einzellichtquellen-Arrays gemäß Figur 3 dargestellt. Es sind zwei Einzellichtquellen an den Positionen 102b und 104b eingeschaltet. Das Licht der Einzellichtquellen wird, wie in Figur 1 dargestellt, durch die Beleuchtungsoptik und das Hauptobjektiv 20 auf dem Fundus 31 des zu beobachtenden Auges 30 abgebildet. Das Auge ist schematisch durch den Kreis 310 dargestellt. Die Abbildung einer Einzellichtquelle auf dem Fundus 31 wird als Beleuchtungsspot bezeichnet. Die Einzellichtquelle an der Position 102b wird koaxial in einen ersten, bzw. linken Beobachtungsstrahlengang eingekoppelt und bildet auf dem Fundus einen ersten Beleuchtungsspot 301. Die Einzellichtquelle an der Position 104b wird koaxial in den zweiten, bzw. rechten Beobachtungsstrahlengang eingekoppelt und bildet auf dem Fundus einen zweiten Beleuchtungsspot 302.

Die auf den Fundus projizierten Abbildungen des jeweils ersten und zweiten Beobachtungsstrahlenganges werden als Beobachtungsspots bezeichnet und sind als gestrichelte Linie dargestellt. Ein erster Beobachtungspot 303 des ersten Beobachtungsstrahlenganges ist größer als der erste Beleuchtungsspot 301 und ein zweiter Beobachtungsspot 304 des zweiten Beobachtungsstrahlengangs ist größer als der zweite Beleuchtungsspot 302. Der erste Beleuchtungsspot 301 liegt vollständig innerhalb des Bereiches des ersten Beobachtungsspots 303. Der zweite Beleuchtungsspot 302 liegt vollständig innerhalb des zweiten Beobachtungsspots 304. Bei einer ersten Beleuchtungsvariante 110 gemäß Figur 4 ist durch die kleinen Beleuchtungsspots 301, 302 ein sehr guter Kontrast der Phasenobjekte erreichbar.

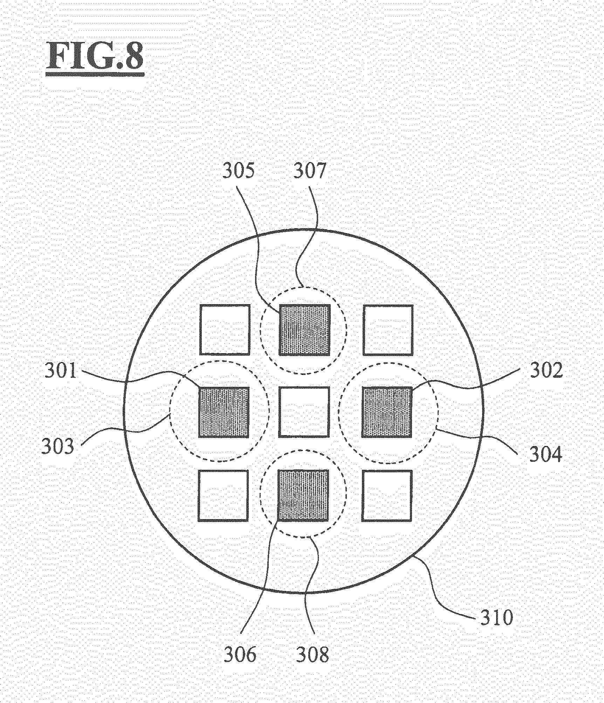

Figur 8 zeigt eine Abbildung der Beleuchtungsspots auf einem Fundus für zwei stereoskopische Beobachter. Ein zweiter Beobachter sieht den Fundus gegenüber dem ersten Beobachter in einer um 90° versetzten Position.

Wie bereits in Figur 7 beschrieben, sind für den ersten Beobachter die Abbildungen der Beleuchtungsspots auf dem Fundus und die zugehörigen Beobachtungsspots für eine erste Stereokoaxial-Beleuchtung dargestellt.

Figur 8 unterscheidet sich von der Beleuchtungssituation gemäß Figur 7 dadurch, dass zusätzlich eine zweite Stereokoaxial-Beleuchtung für einen zweiten Beobachter in 90°-Position in Bezug auf den ersten Beobachter vorhanden ist. Die Einzellichtquelle an der Position 103a wird koaxial in den linken Beobachtungsstrahlengang für den zweiten Beobachter eingekoppelt und bildet auf dem Fundus einen dritten Beleuchtungsspot 305. Die Einzellichtquelle an der Position 103c wird koaxial in den rechten Beobachtungsstrahlengang für den zweiten Beobachter eingekoppelt und bildet auf dem Fundus einen vierten Beleuchtungsspot 306. Der dritte Beleuchtungsspot liegt innerhalb eines dritten Beobachtungspots 307 für einen linken Beobachtungsstrahlengang des zweiten Beobachters. Der vierte Beleuchtungsspot 306 liegt innerhalb eines vierten Beobachtungspots 308 für einen rechten Beobachtungsstrahlengang des zweiten Beobachters.

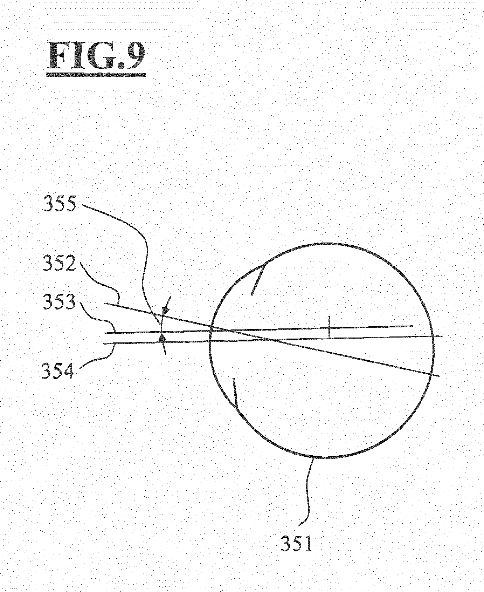

Figur 9 zeigt ein Auge, das auf ein Fixierlicht ausgerichtet ist. Bei der Anwendung eines Operationsmikroskops in der Ophthalmochirurgie, beispielsweise bei der Kataraktchirurgie oder der refraktiven Hornhautchirurgie ist es oft notwendig das Auge bezüglich eines Beobachtungsstrahlenganges oder der optischen Achse eines Messsystems auszurichten. Solche Messsysteme können beispielsweise ein Keratoskop oder ein Messsystem zur intraoperativen Refraktionsmessung sein. Dabei soll die Sehachse eines zu behandelnden Auges zu der optischen Achse eines Beobachtungsstrahlenganges oder eines Messsystems ausgerichtet sein.

Ein Auge 351 hat eine optische Achse 352 und eine Sehachse 354, die unterschiedlich im Auge 351 verlaufen können. Ebenso verläuft die Sehachse 354 nicht zwingend durch die Pupillenmitte. Die Ausrichtung des Auges 351 kann dadurch erfolgen, dass dem Auge 351 ein Fixierlicht als Fixationspunkt angeboten wird. Ist das Auge auf das Fixierlicht ausgerichtet, ist eine Fixierlinie 353 definiert, die parallel zur Sehachse 354 verläuft. Die Fixierlinie 353 kann dabei einen Winkel 355 bezüglich der optischen Achse 352 des Auges 351 einschließen. Bei einer Ausrichtung auf den Fixationspunkt befindet sich das Auge 351 in einer definierten Position.

Wird ein Fixierlicht als zusätzliche Lichtquelle mittels eines Trägers an einem Operationsmikroskop angeordnet, beeinflusst dies nachteilig den freien Arbeitsabstand zwischen dem Hauptobjektiv des Operationsmikroskops und dem Auge.

Das Fixierlicht kann jedoch durch eine Einzellichtquelle des Lichtquellenarrays 3 ausgeführt sein. Das Fixierlicht kann, wie in der dritten Beleuchtungsvariante 112 gemäß Figur 4 dargestellt, beispielsweise durch die zentrale Einzellichtquelle an der Position 103b angeordnet sein. Das Fixierlicht liegt dann beispielsweise in der Mitte zwischen dem linken und dem rechten Beobachtungsstrahlengang. Die zentrale Einzellichtquelle kann dazu in einer anderen Farbe, beispielsweise rot, ausgeführt sein. Für das Fixierlicht ist kein weiteres optisches Element im Beleuchtungslichtstrahlengang notwendig. Ein rechtssichtiges Auge sieht das Fixierlicht scharf. Es ist auch vorstellbar, zusätzliche Einzellichtquellen als Fixierlicht zwischen den als Beleuchtungslichtquellen eingesetzten Einzellichtquellen anzuordnen.

## Bezugszeichenliste

| | | | |
|---|---|---|---|
| 1 | B eleuchtungsvorrichtung | 55 | Zweiter Steckverbinder |
| 2 | Lichtquelleneinheit | 56 | Erste Aussparung |
| 3 | Einzellichtquellen-Array | 57 | Zweite Aussparung |
| 3a | Erste Einzellichtquelle | | |
| 3b | Zweite Einzellichtquelle | 100 | Einzellichtquellen-Array |
| 3c | Dritte Einzellichtquelle | 101 | Erste Spalte |
| 4 | Optische Achse des Beleuchtungsstrahlenganges | 102 | Zweite Spalte |
| | | 103 | Dritte Spalte |
| 5 | Steuerungsvorrichtung | 104 | Vierte Spalte |
| 6 | Leitung | 105 | Fünfte Spalte |
| 7 | Leitung | a | Erste Zeile |
| 10 | Erste Linsengruppe | b | Zweite Zeile |
| 11 | Zweite Linsengruppe | c | Dritte Zeile |
| 13 | Leuchtfeldblende | | |
| 14 | Blendeneinrichtung | 110 | Erste Beleuchtungsvariante |
| 15a | Erster Beleuchtungsstrahlenbündel | 111 | Zweite Beleuchtungsvariante |
| 15b | Zweites Beleuchtungsstrahlenbündel | 112 | Dritte Beleuchtungsvariante |
| 15c | Drittes Beleuchtungsstrahlenbündel | 113 | Vierte Beleuchtungsvariante |
| 20 | Hauptobjektiv | 114 | Fünfte Beleuchtungsvariante |
| 21 | Optische Achse des Beobachtungsstrahlenganges | 115 | Sechste Beleuchtungsvariante |
| | | 116 | Siebte Beleuchtungsvariante |
| 22 | Umlenkelement | 117 | Achte Beleuchtungsvariante |
| 23 | Beobachtungsoptik | | |
| 24 | Kamera | 200 | Blendeneinrichtung |

(fortgesetzt)

| | | | | |
|---|---|---|---|---|
| 30 | Auge | 201 | Erste Spalte |
| 31 | Fundus | 202 | Zweite Spalte |
| | | 203 | Dritte Spalte |
| 50 | Lichtquelleneinheit | 204 | Vierte Spalte |
| 51 | Platine | 205 | Fünfte Spalte |
| 52 | LED-Matrix | a' | Erste Zeile |
| 53 | Flachbandkabel | b' | Zweite Zeile |
| 54 | Erster Steckverbinder | c' | Dritte Zeile |
| 210 | Blendenöffnungen | | |
| 301 | Erster Beleuchtungsspot | | |
| 302 | Zweiter Beleuchtungsspot | | |
| 303 | Erster Beobachtungsspot | | |
| 304 | Zweiter Beobachtungsspot | | |
| 305 | Dritter Beleuchtungsspot | | |
| 306 | Vierter Beleuchtungsspot | | |
| 307 | Dritter Beobachtungsspot | | |
| 308 | Vierter Beobachtungsspot | | |
| 310 | Kreis | | |
| 351 | Auge | | |
| 352 | Optische Achse des Auges 351 | | |
| 353 | Fixierlinie | | |
| 354 | Sehachse | | |
| 355 | Winkel | | |

**Patentansprüche**

1. Operationsmikroskop mit einer Beleuchtungsvorrichtung (1), zur Einstellung unterschiedlicher Beleuchtungsvarianten, umfassend

   eine Beleuchtungslichtquelle (2, 50) mit unabhängig voneinander steuerbaren Einzellichtquellen (3a, 3b, 3c), die in einer ersten Ebene in einem zweidimensionalen Array (3, 100) angeordnet sind, und
   einen Beleuchtungsstrahlengang mit einer Beleuchtungsoptik (10) und einer optischen Achse (4),
   wobei durch die Beleuchtungsoptik (10) eine zu der ersten Ebene konjugierte Ebene gebildet ist,
   wobei eine Blendeneinrichtung (14, 200) in der zu der ersten Ebene konjugierten Ebene angeordnet ist, wobei die Blendeneinrichtung (14, 200) eine Anzahl von Blendenöffnungen (210) aufweist, wobei jede Blendenöffnung (210) einer Einzellichtquelle (3a, 3b, 3c) zugeordnet ist,
   wobei Größe und Form der Blendenöffnungen (210) an Bilder der Einzellichtquellen in der konjugierten Ebene angepasst sind,
   wobei eine erste Hauptebene (H) und eine zweite Hauptebene (H') durch die Beleuchtungsoptik (10) definiert ist,
   wobei ein erster Abstand s als Abstand zwischen der ersten Hauptebene (H) und der ersten Ebene und ein zweiter Abstand s' als Abstand zwischen der zweiten Hauptebene (H') und der zu der ersten Ebene konjugierten Ebene gebildet ist, wobei für einen Vergrößerungsmaßstab folgende Beziehung erfüllt ist:

   $$1 \leq \left| \frac{s'}{s} \right| \leq 4$$

2. Operationsmikroskop nach Anspruch 1,
   **dadurch gekennzeichnet, dass**
   für den Vergrößerungsmaßstab folgende Beziehung erfüllt ist:

$$1.3 \leq \left| \frac{s'}{s} \right| \leq 3$$

3. Operationsmikroskop nach Anspruch 1,
**dadurch gekennzeichnet, dass**
für den Vergrößerungsmaßstab folgende Beziehung erfüllt ist:

$$1.6 \leq \left| \frac{s'}{s} \right| \leq 2.3$$

4. Operationsmikroskop nach einem der Ansprüche 1 bis 3,
**dadurch gekennzeichnet, dass**
die Beleuchtungslichtquelle (2, 50) mindestens vier Einzellichtquellen aufweist.

5. Operationsmikroskop nach einem der Ansprüche 1 bis 4,
**dadurch gekennzeichnet, dass**
in dem zweidimensionalen Array (3, 100) nicht alle Positionen durch Einzellichtquellen besetzt sind.

6. Operationsmikroskop nach einem der bisherigen Ansprüche,
**dadurch gekennzeichnet, dass**
die Einzellichtquellen (3a, 3b, 3c) quadratisch, rechteckig oder rund ausgeführt sind.

7. Operationsmikroskop nach einem der bisherigen Ansprüche,
**dadurch gekennzeichnet, dass**
die in dem zweidimensionalen Array (3, 100) angeordneten Einzellichtquellen kreuzförmig angeordnet sind.

8. Operationsmikroskop nach einem der Ansprüche 1 bis 6,
**dadurch gekennzeichnet, dass**
die in dem zweidimensionalen Array (3, 100) angeordneten Einzellichtquellen in mindestens zwei Zeilen und mindestens zwei Spalten angeordnet sind.

9. Operationsmikroskop nach einem der bisherigen Ansprüche,
**dadurch gekennzeichnet, dass**
eine Steuerungsvorrichtung (5) vorhanden ist, durch die die Helligkeit jeder Einzellichtquelle durch Spannungs- und/oder Stromsteuerung oder durch eine Pulsweitenmodulation mit frei einstellbaren Pulsverhältnissen steuerbar ist.

10. Operationsmikroskop nach Anspruch 9,
**dadurch gekennzeichnet, dass**
die Steuerungsvorrichtung (5) derart ausgebildet ist, dass eine oder mehrere Einzellichtquellen mit geringerer oder höherer Intensität oder mit einem anderen Frequenzmuster als die übrigen Einzellichtquellen ansteuerbar sind, so dass diese eine oder mehrere Einzellichtquellen ein Fixierlicht bilden.

11. Operationsmikroskop nach Anspruch 9 oder 10,
**dadurch gekennzeichnet, dass**
eine oder mehrere Einzellichtquellen zur Abstrahlung von Licht einer anderen Wellenlänge als die übrigen Einzellichtquellen ausgebildet ist.

12. Operationsmikroskop nach einem der bisherigen Ansprüche,
**dadurch gekennzeichnet, dass**

in einem Beobachtungsstrahlengang (23) eine Kamera (24) angeordnet ist, die mit einer Steuerungsvorrichtung (5) verbunden ist,
wobei die Steuerungsvorrichtung (5) zur Detektion eines Rotreflexes eines Auges (31, 351) und zur Ansteuerung der in dem zweidimensionalen Array (3, 100) angeordneten Einzellichtquellen in Abhängigkeit von einer Maßzahl für den Rotreflex ausgebildet ist.

**13.** Verfahren zur automatischen Umschaltung einer Beleuchtungsvariante eines Operationsmikroskops nach Anspruch 12,
**gekennzeichnet durch die Schritte:**

- Aufnahme eines Bildes eines Auges (31, 351) durch die in dem Beobachtungsstrahlengang angeordnete Kamera (24);
- Auswerten des Bildes der Kamera (24) durch die Steuerungsvorrichtung (5);
- Detektieren eines Rotreflexes des Auges (31, 351) durch die Steuerungsvorrichtung (5);
- Bestimmen einer Maßzahl für den Rotreflex;
- Ansteuern der in einem zweidimensionalen Array (3, 100) angeordneten Einzellichtquellen in Abhängig der Maßzahl für den Rotreflex.

**Claims**

**1.** Surgical microscope having an illumination device (1) for setting different illumination variants, comprising

an illumination light source (2, 50) having mutually independently controllable individual light sources (3a, 3b, 3c) arranged in a first plane in a two-dimensional array (3, 100), and
an illumination beam path having an illumination optical unit (10) and an optical axis (4),
wherein a plane conjugate with respect to the first plane is formed by the illumination optical unit (10),
wherein an aperture device (14, 200) is arranged in the plane conjugate with respect to the first plane, wherein the aperture device (14, 200) has a number of aperture openings (210), wherein each aperture opening (210) is assigned to an individual light source (3a, 3b, 3c),
wherein size and shape of the aperture openings (210) are adapted to images of the individual light sources in the conjugate plane,
wherein a first principal plane (H) and a second principal plane (H') are defined by the illumination optical unit (10),
wherein a first distance s is formed as distance between the first principal plane (H) and the first plane and a second distance s' is formed as distance between the second principal plane (H') and the plane conjugate with respect to the first plane, wherein the following relationship is fulfilled for a magnification scale:

$$1 \leq \left| \frac{s'}{s} \right| \leq 4,$$
.

**2.** Surgical microscope according to Claim 1, **characterized in that**
the following relationship is fulfilled for the magnification scale:

$$1.3 \leq \left| \frac{s'}{s} \right| \leq 3,$$
.

**3.** Surgical microscope according to Claim 1, **characterized in that**
the following relationship is fulfilled for the magnification scale:

$$1.6 \leq \left| \frac{s'}{s} \right| \leq 2.3$$
.

**4.** Surgical microscope according to any of Claims 1 to 3,
**characterized in that**
the illumination light source (2, 50) has at least four individual light sources.

**5.** Surgical microscope according to any of Claims 1 to 4,
**characterized in that**
not all positions in the two-dimensional array (3, 100) are occupied by individual light sources.

**6.** Surgical microscope according to any of the preceding claims,

**EP 2 960 705 B1**

**characterized in that**
the individual light sources (3a, 3b, 3c) are embodied in square, rectangular or round fashion.

7. Surgical microscope according to any of the preceding claims,
**characterized in that**
the individual light sources arranged in the two-dimensional array (3, 100) are arranged in a cruciform fashion.

8. Surgical microscope according to any of Claims 1 to 6,
**characterized in that**
the individual light sources arranged in the two-dimensional array (3, 100) are arranged in at least two rows and at least two columns.

9. Surgical microscope according to any of the preceding claims,
**characterized in that**
a control device (5) is present, by which the brightness of each individual light source is controllable by voltage and/or current control or by a pulse width modulation with freely settable pulse ratios.

10. Surgical microscope according to Claim 9,
**characterized in that**
the control device (5) is embodied in such a way that one or a plurality of individual light sources are drivable with lower or higher intensity or with a different frequency pattern than the rest of the individual light sources, such that said one or said plurality of individual light sources form a fixation light.

11. Surgical microscope according to Claim 9 or 10,
**characterized in that**
one or a plurality of individual light sources are designed for emitting light having a different wavelength than the rest of the individual light sources.

12. Surgical microscope according to any of the preceding claims,
**characterized in that**

a camera (24) is arranged in an observation beam path (23), said camera being connected to a control device (5), wherein the control device (5) is designed for detecting a red reflex of an eye (31, 351) and for driving the individual light sources arranged in the two-dimensional array (3, 100) in a manner dependent on a dimension figure for the red reflex.

13. Method for automatically switching an illumination variant of a surgical microscope according to Claim 12,
**characterized by the following steps:**

- recording an image of an eye (31, 351) by means of the camera (24) arranged in the observation beam path;
- evaluating the image from the camera (24) by means of the control device (5);
- detecting a red reflex of the eye (31, 351) by means of the control device (5);
- determining a dimension figure for the red reflex;
- driving the individual light sources arranged in a two-dimensional array (3, 100) in a manner dependent on the dimension figure for the red reflex.


**Revendications**

1. Microscope chirurgical équipé d'un dispositif d'éclairage (1) destiné à régler différentes variantes d'éclairage et comprenant

une source de lumière d'éclairage (2, 50) pourvue de sources de lumière individuelles (3a, 3b, 3c) qui peuvent être commandées indépendamment les unes des autres et qui sont disposées dans un premier plan suivant un réseau bidimensionnel (3, 100), et
un trajet de faisceau d'éclairage comprenant une optique d'éclairage (10) et un axe optique (4),
un plan conjugué au premier plan étant formé par l'optique d'éclairage (10),
un module à diaphragme (14, 200) étant disposé dans le plan conjugué au premier plan, le module à diaphragme (14, 200) comportant un certain nombre d'ouvertures de diaphragme (210), chaque ouverture de diaphragme (210) étant associée à une source de lumière individuelle (3a, 3b, 3c),

la taille et la forme des ouvertures de diaphragme (210) étant adaptées à des images des sources de lumière individuelles dans le plan conjugué,

un premier plan principal (H) et un deuxième plan principal (H') étant définis par l'optique d'éclairage (10),

une première distance s étant formée comme la distance entre le premier plan principal (H) et le premier plan et une deuxième distance s' étant formée comme la distance entre le deuxième plan principal (H') et le plan conjugué au premier plan, la relation suivante étant satisfaite pour une échelle de grossissement :

$$1 \leq \left| \frac{s'}{s} \right| \leq 4$$

2. Microscope chirurgical selon la revendication 1,
   **caractérisé en ce que**
   la relation suivante est satisfaite pour l'échelle de grossissement :

$$1{,}3 \leq \left| \frac{s'}{s} \right| \leq 3$$

3. Microscope chirurgical selon la revendication 1,
   **caractérisé en ce que**
   la relation suivante est satisfaite pour l'échelle de grossissement :

$$1{,}6 \leq \left| \frac{s'}{s} \right| \leq 2{,}3$$

4. Microscope chirurgical selon l'une des revendications 1 à 3,
   **caractérisé en ce que**
   la source de lumière d'éclairage (2, 50) comporte au moins quatre sources de lumière individuelles.

5. Microscope chirurgical selon l'une des revendications 1 à 4,
   **caractérisé en ce que**
   dans le réseau bidimensionnel (3, 100), les positions ne sont pas toutes occupées par des sources lumineuses individuelles.

6. Microscope chirurgical selon l'une des revendications précédentes,
   **caractérisé en ce que**
   les sources lumineuses individuelles (3a, 3b, 3c) sont carrées, rectangulaires ou rondes.

7. Microscope chirurgical selon l'une des revendications précédentes,
   **caractérisé en ce que**
   les sources de lumière individuelles disposées dans le réseau bidimensionnel (3, 100) sont disposées en forme de croix.

8. Microscope chirurgical selon l'une des revendications 1 à 6,
   **caractérisé en ce que**
   les sources de lumière individuelles disposées dans le réseau bidimensionnel (3, 100) sont disposées sur au moins deux rangées et au moins deux colonnes.

9. Microscope chirurgical selon l'une des revendications précédentes,
   **caractérisé en ce que**
   un dispositif de commande (5) est prévu qui permet de commander la luminosité de chaque source de lumière individuelle par le biais d'une commande de tension et/ou de courant ou par le biais d'une modulation de largeur d'impulsion avec des rapports d'impulsion librement réglables.

**10.** Microscope chirurgical selon la revendication 9,
**caractérisé en ce que**
le dispositif de commande (5) est conçu de manière à ce qu'une ou plusieurs sources de lumière individuelles puissent être commandées avec une intensité plus faible ou plus élevée ou avec un modèle de fréquence différent de celui des autres sources de lumière individuelles de sorte que ces une ou plusieurs sources de lumière individuelles forment une lumière de fixation.

**11.** Microscope chirurgical selon la revendication 9 ou 10,
**caractérisé en ce que**
une ou plusieurs sources de lumière individuelles sont conçues pour émettre une lumière d'une longueur d'onde différente de celle des autres sources de lumière individuelles.

**12.** Microscope chirurgical selon l'une des revendications précédentes,
**caractérisé en ce que**

une caméra (24) est disposée dans un trajet de faisceau d'observation (23), laquelle caméra est reliée à un dispositif de commande (5),
le dispositif de commande (5) étant conçu pour détecter un reflet rouge d'un œil (31, 351) et pour commander les sources de lumière individuelles, disposées dans le réseau bidimensionnel (3, 100), en fonction d'une mesure du reflet rouge.

**13.** Procédé de commutation automatique d'une variante d'éclairage d'un microscope chirurgical selon la revendication 12,
**caractérisé par** les étapes suivantes :

- acquérir une image d'un œil (31, 351) par le biais de la caméra (24) disposée dans le trajet de faisceau d'observation ;
- évaluer l'image issue de la caméra (24) par le biais du dispositif de commande (5) ;
- détecter un réflexe rouge de l'œil (31, 351) par le biais du dispositif de commande (5) ;
- déterminer une mesure du réflexe rouge ;
- commander les sources de lumière individuelles, disposées dans un réseau bidimensionnel (3, 100), en fonction de la mesure du réflexe rouge.

# FIG.1

# FIG.2

# FIG.3

# FIG.4

# FIG.5

# FIG.6

# FIG.7

301
302
303
304
310

# FIG.8

FIG.9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP H0868942 A
- US 2012057013 A1
- US 2010302630 A1
- DE 10304267 A1
- DE 19856847 A1
- DE 102012217967 A1
- DE 102006013761 A1